# EUROPEAN PATENT SPECIFICATION

(11) **EP 3 373 903 B1**
(45) Date of publication and mention of the grant of the patent: **08.03.2023**
(21) Application number: 15908092.8
(22) Date of filing: 13.11.2015
(51) Int. Cl.: A61K 8/81, A61K 8/27, A61K 8/24, A61K 8/21, A61K 8/19, A61Q 11/00

(54) **DENTIFRICE COMPOSITIONS WITH ANTI-TARTAR AND ANTI-BACTERIAL BENEFITS**
ZAHNPASTAZUSAMMENSETZUNGEN MIT WIRKUNG GEGEN ZAHNSTEIN UND BAKTERIEN
COMPOSITIONS DENTIFRICE AYANT DES EFFETS BÉNÉFIQUES ANTI-TARTRE ET ANTI-BACTÉRIEN

(43) Date of publication of application: 19.09.2018
(73) Proprietor: The Procter & Gamble Company, Cincinnati, OH 45202 (US)
(72) Inventor: SHANMUGAM, Thanigaivel, Beijing 101312 (CN); STRAND, Ross, Singapore 138547 (SG)
(74) Representative: P&G Patent Germany
(86) International application number: PCT/CN2015/094514
(87) International publication number: WO 2017/079962

(56) References cited:
- WO-A1-2006/012967
- WO-A1-2006/065403
- WO-A1-2009/134657
- WO-A1-2014/088575
- WO-A1-2014/100928
- WO-A2-2007/076444
- US-A1- 2007 053 849
- US-A1- 2014 127 143

## Description

### FIELD OF THE INVENTION

The present invention relates to certain zinc containing dentifrice compositions having improved anti-tartar or anti-bacterial benefits.

### BACKGROUND OF THE INVENTION

Dentifrice compositions are well known for dental and oral hygiene care. High water (e.g., >44 wt%) and high carbonate (e.g., >24 wt%) formulation chassis are cost effective for many markets and consumers. These compositions are formulated at relatively high pH (e.g., pH 8-11) for many reasons including fluoride stability (e.g., sodium monofluorophosphate). Soluble zinc ions are reported to have anti-tartar and anti-bacterial benefits. However, at relatively high pH solubilizing zinc proves challenging given, for example, that insoluble zinc oxide is typically formed at the pH conditions. For example, WO2006/012967A1 discloses to stabilize zinc ions with citrate, wherein the molar ratio from zinc to citrate is best from 1:1.3 to 1:1.7. Accordingly there is a need to improve the solubilization of zinc ions in high water (e.g., >44 wt%), high carbonate (e.g., >24 wt%), alkaline dentifrice compositions to provide anti-tartar or anti-bacterial benefits.

### SUMMARY OF THE INVENTION

A surprising discovery is the positive role of polymethyl vinyl ether maleic anhydride copolymer on zinc solubility in high water, high carbonate, fluoride ion source, alkaline dentifrice formulations. Polyphosphate also has positive role in these results.

One advantage of the present invention is improved soluble zinc ion availability.

Another advantage is anti-bacterial benefits to teeth or portions of an oral cavity.

Another advantage is anti-tartar benefits to teeth.

Another advantage is the fluoride ion stability.

One aspect of the invention provides for a dentifrice composition comprising: 50% to 60%, by weight of the composition, of water; 27% to 47%, by weight of the composition, of a calcium-containing abrasive comprising calcium carbonate; 0.0025% to 2%, by weight of the composition, of a fluoride ion source (e.g., sodium monofluorophosphate); 0.01% to 10%, by weight of the composition, of a zinc ion source; 0.001% to 5%, by weight of the composition, of a copolymer of maleic anhydride and methyl vinyl ether; a polyphosphate, wherein the polyphosphate is a pyrophosphate; and wherein said composition has a pH greater than 8. Preferably the composition comprises from 0.1% to 15%, by weight of the composition, of the polyphosphate.

These and other features, aspects, and advantages of the present invention will become evident to those skilled in the art from the detailed description which follows.

### DETAILED DESCRIPTION OF THE INVENTION

### Definitions

The term "comprising" as used herein means that steps and ingredients other than those specifically mentioned can be added. This term encompasses the terms "consisting of" and "consisting essentially of." The compositions of the present invention can comprise, consist of, and consist essentially of the essential elements and limitations of the invention described herein, as well as any of the additional or optional ingredients, components, steps, or limitations described herein.

The term "dentifrice" as used herein means paste, gel, powder, tablets, or liquid formulations, unless otherwise specified, that are used to clean the surfaces of the oral cavity. Preferably the dentifrice compositions of the present invention are single phase compositions. The term "teeth" as used herein refers to natural teeth as well as artificial teeth or dental prosthesis.

All percentages, parts and ratios are based upon the total weight of the compositions of the present invention, unless otherwise specified. All such weights as they pertain to listed ingredients are based on the active level and, therefore do not include solvents or by-products that may be included in commercially available materials, unless otherwise specified. The term "weight percent" may be denoted as "wt%" herein. All molecular weights as used herein are weight average molecular weights expressed as grams/mole, unless otherwise specified.

As used herein, the articles including "a" and "an" when used in a claim, are understood to mean one or more of what is claimed or described.

As used herein, the terms "comprise", "comprises", "comprising", "include", "includes", "including", "contain", "contains", and "containing" are meant to be non-limiting, i.e., other steps and other sections which do not affect the end of result can be added. The above terms encompass the terms "consisting of' and "consisting essentially of'.

As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

### Zinc

The dentifrice compositions of the present invention comprise a zinc ion source. Non-limiting examples of a zinc ion source include, but are not limited, to the following: zinc citrate, zinc lactate, zinc tartate, zinc pyrophosphate, zinc maleate, zinc chloride, and combinations thereof. A preferred zinc ion source is zinc chloride. Typically levels of the zinc ion source in the dentifrice compositions of the present invention are from 0.01% to 10%, preferably from 0.1% to 7%, more preferably from 0.2% to 5%, yet more preferably from 0.3% to 3.5%, by weight of the composition. Alternatively the zinc ion (from the zinc ion source) is in the dentifrice composition at level from 400 parts per million (ppm) to 20,000 ppm, preferably 500 parts per million (ppm) to 15,000 ppm, preferably from 1,000 ppm to 6,500 ppm, alternatively from 2,000 ppm to 3,000 ppm.

### Copolymer

The dentifrice composition of the present invention comprises a copolymer of maleic anhydride and methyl vinyl ether, preferably wherein the copolymer is free of monomers and solvents. The zinc ion source / copolymer combination appears to be a factor in increasing the solubility of the zinc ion source in the dentifrice compositions of the present invention.

Preferably the molecular weight of the copolymer of maleic anhydride and methyl vinyl ether is relatively high. The molecular weight of the copolymer is defined by its specific viscosity. In turn, the specific viscosity is determined by dissolving 0.5 g of the copolymer in 50 ml of methyl ethyl ketone and measuring the specific viscosity at 25° C by a viscosimeter. A suitable viscosimeter is a CANNON FENSKE viscosimeter, capillary No. 100. The specific viscosity range of the copolymers of the present invention may include those from 1 to 5, preferably from 2 to 4.5, alternatively from 2.1 to 4.3. Such polymers have been described, for example, in U.S. Pat. No. 5,047,490, column 3, line 35 to column 4, line 39. More preferably the copolymer is 2-Butenedioic acid (2Z)-, polymer with methoxyethene, generally having the formula (C₄H₄O₄·C₃H₆O)ₓ. Such a copolymer is commercially available from ISP CHEMICALS LLC, Calvert City, KY, USA under the tradename GANTREZ^{®} S97^{™}. Typically levels of the copolymer are from 0.001% to 5%, preferably from 0.01% to 4%, preferably from 0.1% to 3%, more preferably from 0.2% to 2%, yet more preferably from 0.3% to 1%, by weight of the composition, of the copolymer.

The copolymer of maleic anhydride and methyl vinyl is preferably hydrolyzed to form a dicarboxylic acid, which is then complexed with the aforementioned zinc ion source. The use of a complexing agent may also be employed. In such a case, the zinc ion source is co-dissolved with the complexing agent. Non-limiting examples of such complexing agents include sodium gluconate, maleic acid, aspartic acid, gluconic acid, succinic acid, glucuronic acid, sodium glutamate, fumaric acid, and combinations thereof.

Preferably the dentifrice compositions of the present have a weight ratio of the zinc ion source to the copolymer of maleic anhydride and methyl vinyl ether, from 1:4 to 4:1; preferably from 1:3 to 3:1; more preferably from 1:2 to 2:1.

### Polyphosphate

The dentifrice compositions of the present invention further comprise a polyphosphate which is pyrophosphate. The polyphosphate in combination with the zinc ion source-copolymer complex appears to be a factor in further increasing the solubility of the zinc ion source in the dentifrice compositions of the present invention. This is particularly true given the alkaline pH of the dentifrice composition of the present invention. Without wishing to be bound by theory, the polyphosphate may help in increasing the zinc ion source-copolymer complex formation (which in turn help facilitate the solubility of the zinc ion).

Polyphosphates are salts or esters of polymeric oxyanions formed from phosphate structural units linked together by sharing oxygen atoms. The polyphosphate of the present invention is a pyrophosphate. In turn, non-limiting examples of pyrophosphate include calcium pyrophosphate, tetrasodium pyrophosphate, disodium pyrophosphate, tetrapotassium pyrophosphate, and combinations thereof. A preferred pyrophosphate is tetrasodium pyrophosphate. The presence of polyphosphate ostensibly increases the amount of soluble zinc ion in the dentifrice compositions of the present invention. Typically levels of the polyphosphate are from 0.1% to 15%, preferably 0.3% to 10%, more preferably from 0.5% to 8%, yet more preferably from 0.9% to 6%, yet still more preferably from 1% to 5%, yet still more preferably from 2% to 4%, alternatively from 0.3% to 6%, by weight of the composition, of the polyphosphate, wherein the polyphosphate is a pyrophosphate, more preferably wherein the pyrophosphate is tetrasodium pyrophosphate. One advantage provided by the use of pyrophosphate is anti-plaque benefits given its calcium chelating thereby mitigating plaque formation. The use of pyrophosphate may also provide the additional benefit of monofluorophosphate stabilization (in those formulations containing monofluorophosphate).

### Water

The dentifrice compositions of the present invention comprise herein from 50% to 60%, by weight of the composition, of water. The water may be added to the formulation and/or may come into the composition from the inclusion of other ingredients. Preferably the water is USP water.

### Calcium-containing abrasive

The compositions of the present invention comprise from 27% to 47%, by weight of the composition, of a calcium-containing abrasive, wherein the calcium-containing abrasive comprises calcium carbonate.

Preferably, the composition comprises from 27% to 37%, more preferably from 28% to 34%, even more preferably from 29% to 33%, by weight of the composition, alternatively combinations thereof, of a calcium-containing abrasive.

Preferably, the calcium-containing abrasive is calcium carbonate. More preferably, the calcium-containing abrasive is selected from the group consisting of fine ground natural chalk, ground calcium carbonate, precipitated calcium carbonate, and combinations thereof.

Fine ground natural chalk (FGNC) is one of the more preferred calcium-containing abrasives useful in the present invention. It is obtained from limestone or marble. FGNC may also be modified chemically or physically by coating during milling or after milling by heat treatment. Typical coating materials include magnesium stearate or oleate. The morphology of FGNC may also be modified during the milling process by using different milling techniques, for example, ball milling, air-classifier milling or spiral jet milling. One example of natural chalk is described in WO 03/030850 having a medium particle size of 1 to 15 µm and a BET surface area of 0.5 to 3 m²/g. The natural calcium carbonate may have a particle size of 325 to 800 mesh, alternatively a mess selected from 325, 400, 600, 800, or combinations thereof; alternatively, the particle size is from 0.1 to 30 microns, or from 0.1 to 20 microns, or from 5 to 20 microns. In one embodiment, the composition comprises from 0% to 5%, preferably 0% to 3%, more preferably 0% to 1%, by weight of the composition, of a silicate; yet more preferably the composition is substantially free silicate.

### Fluoride ion source

The compositions may include an effective amount of an anti-caries agent. In one embodiment, the anti-caries agent is a fluoride ion source. The fluoride ion is present in an amount sufficient to give a fluoride ion concentration in the composition at 25° C, and is used at levels of from 0.0025% to 2.0% by weight of the composition, to provide anti-caries effectiveness. Representative fluoride ion sources include: stannous fluoride, sodium fluoride, potassium fluoride, amine fluoride, sodium monofluorophosphate, and zinc fluoride. In one embodiment the dentifrice composition contains a fluoride source selected from stannous fluoride, sodium fluoride, and mixtures thereof. In one embodiment, the fluoride ion source is sodium monofluorophosphate, and wherein the composition comprises 0.0025% to 2%, by weight of the composition, of the sodium monofluorophosphate, alternatively from 0.5% to 1.5%, alternatively from 0.6% to 1.7%, alternatively combinations thereof.

In one example, the dentifrice compositions of the present invention may have a dual fluoride ion source, specifically sodium monofluorophosphate and an alkaline metal fluoride. Such an approach may provide an improvement in mean fluoride update.

pH The pH of the dentifrice composition is greater than pH 8, more preferably from pH 8.1 to pH 11. Preferably, the pH is greater than 8.1, more preferably the pH is greater than pH 8.5, even more preferably the pH is greater than pH 9, alternatively the pH is from pH 9.0 to pH 10.5, alternatively from pH 8.5 to pH 10. The relatively high pH of the present inventive composition is for fluoride stability. Without wishing to be bound on theory, at below pH 8 calcium ion may bind with the fluoride. Thus, the dentifrice composition has a pH greater than 8.0 to maximize the stability of the fluoride ion source. A method for assessing pH of dentifrice is described is provided the analytical methods section provided below. For purposes of clarification, although the analytical method describes testing the dentifrice composition when freshly prepared, for purposes of claiming the present invention, the pH may be taken at anytime during the product's reasonable lifecycle (including but not limited to the time the product is purchased from a store and brought to the consumer's home).

### pH modifying agent

The dentifrice compositions herein may include an effective amount of a pH modifying agent, alternatively wherein the pH modifying agent is a pH buffering agent. The pH modifying agents, as used herein, refer to agents that can be used to adjust the pH of the dentifrice compositions to the above-identified pH range. The pH modifying agents may include alkali metal hydroxides, ammonium hydroxide, organic ammonium compounds, carbonates, sesquicarbonates, borates, silicates, phosphates, imidazole, and mixtures thereof. Specific pH agents include monosodium phosphate (monobasic sodium phosphate or "MSP"), trisodium phosphate (sodium phosphate tribasic dodecahydrate or "TSP"), sodium benzoate, benzoic acid, sodium hydroxide, potassium hydroxide, alkali metal carbonate salts, sodium carbonate, imidazole, sodium gluconate, lactic acid, sodium lactate, citric acid, sodium citrate, phosphoric acid. In one embodiment, 0.01% to 3%, preferably from 0.1% to 1%, by weight of the composition, of TSP, and 0.001% to 2%, preferably from 0.01% to 0.3%, by weight of the composition, of monosodium phosphate is used. Without wishing to be bound by theory, TSP and monosodium phosphate may also have calcium ion chelating activity and therefore provide some monofluorophosphate stabilization (in those formulations containing monofluorophosphate).

A method for assessing pH of dentifrice is described. The pH is measured by a pH Meter with Automatic Temperature Compensating (ATC) probe. The pH Meter is capable of reading to 0.001 pH unit. The pH electrode may be selected from one of the following (i) Orion Ross Sure-Flow combination: Glass body - VWR #34104-834/Orion #8172BN or VWR#10010-772/Orion #8172BNWP; Epoxy body - VWR #34104-830/Orion #8165BN or VWR#10010-770/Orion #8165BNWP; Semi-micro, epoxy body - VWR #34104-837/Orion #8175BN or VWR#10010-774/Orion #3175BNWP; or (ii) Orion PerpHect combination:VWR #34104-843/Orion #8203BN semi-micro, glass body; or (iii) suitable equivalent. The automatic temperature compensating probe is Fisher Scientific, Cat #13-620-16.

A 25% by weight slurry of dentifrice is prepared with deionized water, and thereafter is centrifuged for 10 minutes at 15,000 rotations-per-minute using a SORVALL RC 28S centrifuge and SS-34 rotor (or equivalent gravitational force, at 24149g force). The pH is assessed in supernatant after one minute or the taking reading is stabilized. After each pH assessment, the electrode is washed with deionized water. Any excess water is wiped with a laboratory grade tissue. When not in issue, the electrode is kept immersed in a pH 7 buffer solution or an appropriate electrode storage solution.

### Low or Free Humectants

The compositions herein may be substantially free or free of humectants, alternatively contain low levels of humectants. The term "humectant," for the purposes of present invention, include edible polyhydric alcohols such as glycerin, sorbitol, xylitol, butylene glycol, propylene glycol, and combinations thereof. In one embodiment, the humectant is a polyol, preferably wherein the polyol is selected from sorbitol, glycerin, and combinations thereof. In yet another embodiment, the humectant is sorbitol. In one embodiment, the composition comprises from 0% to less than 5%, by weight of the composition, of humectants, preferably from 0% to 4%, alternatively from 0% to 3%, alternatively from 0% to 2%, alternatively from 0% to 1%, by weight of th4 composition, of humectants. A potential advantage of having a dentifrice composition that is free or substantially free of humectants is, without wishing to be bound by theory, is those dentifrice compositions that are free of polyols (e.g., glycerin and sorbitol), or have a relatively low amount thereof, may provide better fluoride uptake compared to those compositions having the high levels of such polyols (or humectants for that matter). In one example, the dentifrice compositions of the present invention comprise from 0% to 5%, preferably 0% to 3%, more preferably 0% to 1%, by weight of the composition, of glycerin, sorbitol, or combinations thereof; yet more preferably the composition is substantially free of both glycerin and sorbitol.

### Thickening System

The dentifrice compositions of the present invention may comprise a thickening system. Preferably the dentifrice composition comprises from 0.5% to 4%, preferably from 0.8% to 3.5%, more preferably from 1% to 3%, yet still more preferably from 1.3% to 2.6%, by weight of the composition, of the thickening system. More preferably the thickening system comprises a thickening polymer, a thickening silica, or a combination thereof. Yet more preferably, when the thickening system comprises a thickening polymer, the thickening polymer is selected from a carboxymethyl cellulose, a linear sulfated polysaccharide, a natural gum, and combination thereof. Yet still more preferably, when the thickening system comprises a thickening polymer, the thickening polymer is selected from the group consisting of: (a) 0.01% to 3% of a carboxymethyl cellulose ("CMC") by weight of the composition, preferably 0.1% to 2.5%, more preferably 0.2% to 1.5%, by weight of the composition, of CMC; (b) 0.01% to 2.5%, preferably 0.05 % to 2%, more preferably 0.1% to 1.5%, by weight of the composition, of a linear sulfated polysaccharide, preferably wherein the linear sulfated polysaccharide is a carrageenan; (c) 0.01% to 7%, preferably 0.1% to 4%, more preferably from 0.1 % to 2%, yet more preferably from 0.2% to 1.8%, by weight of the composition, of a natural gum; (d) combinations thereof. Preferably when the thickening system comprises a thickening silica, the thickening silica is from 0.01% to 10%, more preferably from 0.1% to 9%, yet more preferably 1% to 8% by weight of the composition.

Preferably the linear sulfated polysaccharide is a carrageenan (also known as carrageenin). Examples of carrageenan include Kappa-carrageenan, Iota-carrageenan, Lambda-carrageenan, and combinations thereof.

In one example the thickening silica is obtained from sodium silicate solution by destabilizing with acid as to yield very fine particles. One commercially available example is ZEODENT^{®} branded silicas from Huber Engineered Materials (e.g., ZEODENT^{®} 103, 124, 113 115, 163, 165, 167).

In one example the CMC is prepared from cellulose by treatment with alkali and monochloro-acetic acid or its sodium salt. Different varieties are commercially characterized by viscosity. One commercially available example is Aqualon^{™} branded CMC from Ashland Special Ingredients (e.g., Aqualon^{™} 7H3SF; Aqualon^{™} 9M3SF Aqualon^{™} TM9A; Aqualon^{™} TM12A).

Preferably a natural gum is selected from the group consisting of gum karaya, gum arabic (also known as acacia gum), gum tragacanth, xanthan gum, and combination thereof. More preferably the natural gum is xanthan gum. Xanthan gum is a polysaccharide secreted by the bacterium *Xanthomonas camestris.* Generally, xanthan gum is composed of a pentasaccharide repeat units, comprising glucose, mannose, and glucuronic acid in a molar ratio of 2:2:1, respectively. The chemical formula (of the monomer) is C₃₅H₄₉O₂₉. In one example, the xanthan gum is from CP Kelco Inc (Okmulgee, US).

### Viscosity

Preferably the dentifrice compositions of the present invention have a viscosity range from 150,000 centipoise to 850,000 centipoise ("cP"). A method for assessing viscosity is described. The viscometer is Brookfield^{®} viscometer, Model DV-I Prime with a Brookfield "Helipath" stand. The viscometer is placed on the Helipath stand and leveled via spirit levels. The E spindle is attached, and the viscometer is set to 2.5 RPM. Detach the spindle, zero the viscometer and install the E spindle. Then, lower the spindle until the crosspiece is partially submerged in the paste before starting the measurement. Simultaneously turn on the power switch on the viscometer and the helipath to start rotation of the spindle downward. Set a timer for 48 seconds and turn the timer on at the same time as the motor and helipath. Take a reading after the 48 seconds. The reading is in cP.

### PEG

The compositions of the present invention may optionally comprise polyethylene glycol (PEG), of various weight percentages of the composition as well as various ranges of average molecular weights. In one aspect of the invention, the compositions have from 0.01% to 8%, preferably from 0.1% to 5%, more preferably from 0.2% to 4.8%, yet more preferably from 0.3% to 4.2%, yet still more preferably from 0.5% to 4%, by weight of the composition, of PEG. In another aspect of the invention, the PEG is one having a range of average molecular weight from 100 Daltons to 1600 Daltons, preferably from 200 to 1000, alternatively from 400 to 800, alternatively from 500 to 700 Daltons, alternatively combinations thereof. PEG is a water soluble linear polymer formed by the addition reaction of ethylene oxide to an ethylene glycol equivalent having the general formula is: H-(OCH₂CH₂)ₙ-OH. One supplier of PEG is Dow Chemical Company under the brandname of CARBOWAX^{™}. Without wishing to be bound by theory, having some PEG in the dentifrice composition may help with physical stability.

### Sweetener

The oral care compositions herein may include a sweetening agent. These include sweetening agents may include saccharin, dextrose, sucrose, lactose, maltose, levulose, aspartame, sodium cyclamate, D-tryptophan, dihydrochalcones, acesulfame, sucralose, neotame, and mixtures thereof. Sweetening agents are generally used in oral compositions at levels of from 0.005% to 5%, by weight of the composition, alternatively 0.01% to 1%, alternatively from 0.1% to 0.5%, alternatively combinations thereof.

### Surfactant

The dentifrice compositions herein may include a surfactant. The surfactant may be selected from anionic, nonionic, amphoteric, zwitterionic, cationic surfactants, or mixtures thereof. The composition may include a surfactant at a level of from 0.1% to 10%, from 0.025% to 9%, from 0.05% to 5%, from 0.1% to 2.5%, from 0.5% to 2%, or from 0.1% to 1% by weight of the total composition. Non-limiting examples of anionic surfactants may include those described at US 2012/0082630 A1 at paragraphs 32, 33, 34, and 35. Non-limiting examples of zwitterionic or amphoteric surfactants may include those described at US 2012/0082630 A1 at paragraph 36; cationic surfactants may include those described at paragraphs 37 of the reference; and nonionic surfactants may include those described at paragraph 38 of the reference. In one embodiment the composition comprises 0.1% to 5%, preferably 0.1% to 3%, alternatively from 0.3% to 3%, alternatively from 1.2% to 2.4%, alternatively from 1.2% to 1.8%, alternatively from 1.5 % to 1.8%, by weight of the composition, alternatively combinations thereof, of the anionic surfactant sodium lauryl sulfate (SLS).

### Colorant

The compositions herein may include a colorant. Titanium dioxide is one example of a colorant. Titanium dioxide is a white powder which adds opacity to the compositions. Titanium dioxide generally can comprise from 0.25% to 5%, by weight of the composition.

### Flavorant

The compositions herein may include from 0.001% to 5%, alternatively from 0.01% to 4%, alternatively from 0.1% to 3%, alternatively from 0.5% to 2%, alternatively 1% to 1.5%, alternatively 0.5% to 1%, by weight of the composition, alternatively combinations thereof, of a flavorant composition. The term flavorant composition is used in the broadest sense to include flavor ingredients, or sensates, or sensate agents, or combinations thereof. Flavor ingredients may include those described in US 2012/0082630 A1 at paragraph 39; and sensates and sensate ingredients may include those described at paragraphs 40 - 45,. Excluded from the definition of flavorant composition is "sweetener" (as described above).

### EXAMPLES

Four example compositions are prepared and assessed for zinc ion solubility, anti-tartar benefits (by way of crystal growth inhibition), and anti-bacterial benefits (by way of inhibiting lactate formation).

### Compositional Components of Examples 1-4.

**Table 1: Compositional components of examples 1 and 2 (control) and examples 3-4, are provided.**

| Components: (Wt%) | Ex 1 Control | Ex 2 Control | Ex 3 | Ex 4 |
|---|---|---|---|---|
| Water | 55.7 | 54.58 | 53.68 | 52.08 |
| CaCO₃ | 32.0 | 32.0 | 32.0 | 32.0 |
| Sorbitol | 0 | 0 | 0 | 0 |
| Glycerol | 0 | 0 | 0 | 0 |
| 2-Butenedioic acid (2Z)-, polymer with methoxyethene, (GANTREZ^{®} S97^{™}) | 0 | 0.6 | 0.6 | 0.6 |
| ZnCl₂ | 0 | 0.52 | 0.52 | 0.52 |
| Tetra Sodium Pyrophosphate (TSPP) | 0 | 0 | 0.9 | 3.0 |
| Sodium Monofluorophosphate | 1.1 | 1.1 | 1.1 | 1.1 |
| Sodium Caboxymethyl Cellulose | 0.9 | 0.9 | 0.9 | 0.9 |
| Carrageenan | 1.2 | 1.2 | 1.2 | 1.2 |
| Thickener Silica | 2.6 | 2.6 | 2.6 | 2.6 |
| Sodium Lauryl Sulfate | 4.0 | 4.0 | 4.0 | 4.0 |
| Flavor | 0.9 | 0.9 | 0.9 | 0.9 |
| Sodium Monophosphate | 0.2 | 0.2 | 0.2 | 0.2 |
| Sodium Triphosphate | 0.4 | 0.4 | 0.4 | 0.4 |
| Sodium Carbonate | 0.5 | 0.5 | 0.5 | 0.0 |
| Sodium Saccharine | 0.3 | 0.3 | 0.3 | 0.3 |
| Methylparaben | 0.1 | 0.1 | 0.1 | 0.1 |
| Prophlparaben | 0.1 | 0.1 | 0.1 | 0.1 |
| Total: | 100 | 100 | 100 | 100 |

Control example 1 is notably free of GANTREZ^{®} S97^{™} polymer (2-Butenedioic acid (2Z)-, polymer with methoxyethene), ZnCl₂, and tetrasodium pyrophosphate ("TSPP"). Examples 2-4 each have 0.6 wt% of GANTREZ^{®} S97^{™} polymer and 0.53 wt% of ZnCl₂ (2500 parts per million (PPM) of Zn ion). The difference between Examples 2-4 is the amount of TSPP each dentifrice composition contains. Example 2 is free of TSPP, while example 3 has 0.9 wt% of TSPP, and example 4 has the greatest amount at 3.0 wt% of TSPP. All samples are pH buffered between pH 8.5 to pH 10.

### Solubility of Zinc Ion

Insoluble zinc oxide is generally observed at alkaline pH (e.g. pH 8.5 - 10). Zinc solubility in Examples 2-4 is assessed with results provided in Table 2 below. The compositional components of these examples are in Table 1 above.

The method for assessing soluble zinc ion is described. A slurry is prepared from one part example composition and three parts water. The slurry is centrifuged and an aliquot of the supernatant is placed in acid matrix. The solution is analyzed by ICP-OES to assess the amount soluble since in solution (on a weight percentage basis).

Cloudiness is assessed as an indirect way of assessing zinc solubility. The less cloudy a sample, the more likely the zinc is soluble (and less of the insoluble zinc oxide is likely present). Table 2 summarizes the results.

**Table 2 : Cloudiness and the amount of soluble zinc ion are assessed for examples 2-4.**

| **Ex.** | **Components:** | **Concentration (respectively):** | **Cloudiness¹** | **Soluble Zinc (wt%)** |
|---|---|---|---|---|
| 2 | Zn: GANTREZ^{®2} | 2500 PPM³ : 0.6 wt% | +++ | 20.3 |
| 3 | Zn: GANTREZ^{®}:TSPP | 2500 PPM : 0.6 wt% : 0.6 wt% | ++ | 26.9 |
| 4 | Zn: GANTREZ^{®}:TSPP | 2500 PPM : 0.6 wt% :3 wt% | Clear | 68.8 |

| | | | | |
|---|---|---|---|---|
| ¹ Cloudiness increases with +. Therefore + + + has the most cloudiness. ² GANTREZ^{®} S97^{™} ³ Parts Per Million | | | | |

Table 2 indicates that Example 4 is the best performing dentifrice composition by having the least amount of cloudiness thereby suggesting that zinc is likely soluble and the amount of insoluble zinc oxide is minimized. The percentage of soluble zinc also supports this observation given a soluble zinc ion percentage of 68.8 wt%. Notably example 4 has the most amount of TSPP of the examples tested at 3 wt%. Example 3 is the second best performing composition and Example 2 is the least performing composition tested.

### Anti-tartar benefits

The anti-tartar benefits, by way of a Crystal Growth Inhibition (CGI) method, are assessed in a control example 1, and examples 2-4. The results of the CGI method are provided in Table 3 below. The components of these examples are provided in Table 1 above.

The Crystal Growth Inhibition ("CGI") test method is briefly described. The CGI test is designed to examine mineral growth or dissolution kinetics under constant solution pH. The CGI test involves the following steps. A 25% wt/wt composition slurry is prepared and centrifuged at 10,000 rotations per minute ("rpm") for 15 minutes. 10 grams of the resulting supernatant is placed in a clean test tube. 3 ml of a hydroxyapatite (HAP) slurry (about 0.3 g) is added to the supernatant containing test tube, and mixed for 1 minute. 20 g of water is added into the tube to "quench" the reaction. The treatment mixture is centrifuged at 10,000 rpm for 15 minutes and the fluid is decanted. The resulting HAP plug is washed twice by re-suspending in 30 mL of water and centrifuged at 10,000 rpm for 15 minutes. Thereafter the resultant HAP plug is dried in a test tube at 37°C for 24 hours or until dry. The dried HAP plug is ground using a mortar and pestal. 0.050 g of the ground HAP plug is weighed out and injected into a reaction vessel containing 50 mL artificial saliva (1.75 mM calcium, 1.05 mM phosphate and 0.15 M NaCl). The rates of crystal growth are compared against non-inhibited growth curves. The percentage reduction of CGI are provided in table 3 below.

**Table 3: The percentage of CGI reduction verses control are provided.**

| Example | Notable Components (Wt%) | | | % Reduction v. Control |
|---|---|---|---|---|
| | ZnCl₂ | GANTREZ^{®} S97^{™} | TSPP | |
| 1 (Control) | 0 | 0 | 0 | 0 |
| 2 | 0.52 | 0.6 | 0 | 17.5 |
| 3 | 0.52 | 0.6 | 0.6 | 55.6 |
| 4 | 0.52 | 0.6 | 3.0 | 63.4 |

Table 3 indicates that example 4 has higher percentage of CGI reduction (at 63.4%) compared to the other examples and control. Example 3 is the second best performing composition and Example 2 is the least performing composition tested (apart from the control).

### Anti-bacterial benefits

The anti-bacterial benefits, by way of a lactate inhibition method, are assessed in the control example 1 and inventive example 4. The results of the lactate inhibition method are provided in Table 4 below. The components of these examples are provided in Table 1.

The lactate inhibition test method is briefly described. The examples are diluted 30-fold with de-ionized water for testing. Saliva is collected and pooled from 4-6 subjects and concentrated four fold. Medium containing 0.1 TSB and 5% glucose is prepared. 2 ml saliva, 5 ml Medium and 0.5 ml diluted of the respective dentifrice examples are mixed and incubated in 35° C water bath. Microbes present in the saliva ferment glucose to lactate. The less lactate that is produced, the more of anti-bacterial effect is provided. Levels of organic acid are tested at time zero and after two hours and using NMR. Inhibition rate is calculated as 100%- organic acid level of test formula (Ex. 4) /organic acid level of control formula (Ex. 1) * 100%.

**Table 4: The percent inhibition of lactate product is provided between examples 1 (control) and 4.**

| Example | Notable Components | | | % Inhibition rate of lactate production |
|---|---|---|---|---|
| | ZnCl2 (PPM) | GANTREZ^{®} S97^{™} (Wt %) | TSPP (Wt%) | |
| 1 (Control) | 0 | 0 | 0 | 1.92 |
| 4 | 0.52 | 0.6 | 3.0 | 9.10 |

Table 4 suggests that inventive example 4 has antibacterial benefits evidenced by the reduction of lactate production.

The dimensions and values disclosed herein are not to be understood as being strictly limited to the exact numerical values recited. Instead, unless otherwise specified, each such dimension is intended to mean both the recited value and a functionally equivalent range surrounding that value.

## Claims

1. A dentifrice composition comprising:
(a) 50% to 60%, by weight of the composition, of water;
(b) 27% to 47%, by weight of the composition, of a calcium-containing abrasive wherein the calcium-containing abrasive comprises calcium carbonate;
(c) 0.0025% to 2%, by weight of the composition, of a fluoride ion source;
(d) 0.01% to 10%, by weight of the composition, of a zinc ion source;
(e) 0.001% to 5%, by weight of the composition, of a copolymer of maleic anhydride and methyl vinyl ether; and
(f) a polyphosphate, wherein the polyphosphate is a pyrophosphate; and
wherein said composition has a pH greater than 8.

2. The dentifrice composition of claim 1, wherein the copolymer of maleic anhydride and methyl vinyl ether, comprises from 0.01% to 4%, preferably from 0.1% to 3%, more preferably from 0.2% to 2%, yet more preferably from 0.3% to 1%, by weight of the composition.

3. The dentifrice composition of any one of the preceding claims, wherein the copolymer of maleic anhydride and methyl vinyl ether has specific viscosity from 1 to 5, preferably from 2 to 4.5, (according to the method described herein).

4. The dentifrice composition of any one of the preceding claims, wherein the copolymer of maleic anhydride and methyl vinyl ether is the copolymer 2-Butenedioic acid (2Z)-polymer with methoxyethene.

5. The dentifrice composition of any one of the preceding claims, wherein the zinc ion source is selected from the group consisting of zinc citrate, zinc lactate, zinc tartate, zinc pyrophosphate, zinc maleate, zinc chloride, and combinations thereof; preferably the zinc ion source is zinc chloride.

6. The dentifrice composition of any one of the preceding claims, wherein the zinc ion source comprises from 0.1% to 7%, more preferably from 0.2% to 5%, yet more preferably from 0.3% to 3.5%, by weight of the composition.

7. The dentifrice composition of any one of the preceding claims, wherein a zinc ion, from the zinc ion source, comprises a level from 400 parts per million (ppm) to 20,000 ppm, preferably 500 parts per million (ppm) to 15,000 ppm, preferably from 1,000 ppm to 6,500 ppm, alternatively from 2,000 ppm to 3,000 ppm.

8. The dentifrice composition of any one of the preceding claims, wherein the weight ratio of the zinc ion source to the copolymer of maleic anhydride and methyl vinyl ether is from 1:4 to 4:1; preferably from 1:3 to 3:1; more preferably from 1:2 to 2:1.

9. The dentifrice composition of any one of the preceding claims, wherein the pyrophosphate is selected from the group consisting of calcium pyrophosphate, tetrasodium pyrophosphate, disodium pyrophosphate, tetrapotassium pyrophosphate, and combinations thereof; preferably the pyrophosphate is tetrasodium pyrophosphate.

10. The dentifrice composition of claim 9, wherein the pyrophosphate is from 0.1% to 15%, preferably from 0.5% to 8%, more preferably from 0.9% to 6%, yet more preferably from 1% to 5%, by weight of the composition.

11. The dentifrice composition of claim 9 or 10, wherein the zinc ion source is zinc chloride and wherein the copolymer of maleic anhydride and methyl copolymer is 2-Butenedioic acid (2Z)-polymer with methoxyethene.

12. The dentifrice composition of any one of the preceding claims, wherein the zinc ion source and the copolymer of maleic anhydride and methyl vinyl ether form a complex, preferably wherein the copolymer of maleic anhydride and methyl vinyl ether is hydrolyzed.

13. The dentifrice composition of any one of the preceding claims, wherein the fluoride ion source is sodium monofluorophosphate; preferably the composition comprises from 0.2% to 1.5%, preferably from 0.5% to 1 %, more preferably from 0.6% to 0.9%, by weight of the composition, of the sodium monofluorophosphate.

14. The dentifrice composition of any one of the preceding claims, wherein the calcium-containing abrasive comprises 27% to 37%, by weight of the composition, of the calcium carbonate.

15. The dentifrice composition of any one of the preceding claims, wherein the pH is greater than 8.1, preferably greater than pH 8.5, more preferably greater than pH 9, alternatively from pH 8.5 to pH 10.5.

16. The dentifrice composition of any one of the preceding claims, wherein the composition is a single-phase composition.

## Patentansprüche

1. Zahncremezusammensetzung, umfassend:
(a) zu 50 Gew.-% bis 60 Gew.-% der Zusammensetzung Wasser;
(b) zu 27 Gew.-% bis 47 Gew.-% der Zusammensetzung ein calciumhaltiges Schleifmittel, wobei das calciumhaltige Schleifmittel Calciumcarbonat umfasst;
(c) zu 0,0025 Gew.-% bis 2 Gew.-% der Zusammensetzung eine Fluoridionenquelle;
(d) zu 0,01 Gew.-% bis 10 Gew.-% der Zusammensetzung eine Zinkionenquelle;
(e) zu 0,001 Gew.-% bis 5 Gew.-% der Zusammensetzung ein Copolymer aus Maleinsäureanhydrid und Methylvinylether; und
(f) ein Polyphosphat, wobei das Polyphosphat ein Pyrophosphat ist; und
wobei die Zusammensetzung einen pH-Wert über 8 aufweist.

2. Zahncremezusammensetzung nach Anspruch 1, wobei das Copolymer aus Maleinsäureanhydrid und Methylvinylether von 0,01 Gew.-% bis 4 Gew.-%, vorzugsweise von 0,1 Gew.-% bis 3 Gew.-%, mehr bevorzugt von 0,2 Gew.-% bis 2 Gew.-%, noch mehr bevorzugt von 0,3 Gew.-% bis 1 Gew.-% der Zusammensetzung umfasst.

3. Zahncremezusammensetzung nach einem der vorstehenden Ansprüche, wobei das Copolymer aus Maleinsäureanhydrid und Methylvinylether eine spezifische Viskosität von 1 bis 5, vorzugsweise aus 2 bis 4,5 (gemäß dem hierin beschriebenen Verfahren) aufweist.

4. Zahncremezusammensetzung nach einem der vorstehenden Ansprüche, wobei das Copolymer aus Maleinsäureanhydrid und Methylvinylether das Copolymer 2-Butendisäure(2Z)-Polymer mit Methoxyethen ist.

5. Zahncremezusammensetzung nach einem der vorstehenden Ansprüche, wobei die Zinkionenquelle aus der Gruppe ausgewählt ist, bestehend aus Zinkcitrat, Zinklactat, Zinkweinstein, Zinkpyrophosphat, Zinkmaleat, Zinkchlorid und Kombinationen davon; vorzugsweise die Zinkionenquelle Zinkchlorid ist.

6. Zahncremezusammensetzung nach einem der vorstehenden Ansprüche, wobei die Zinkionenquelle von 0,1 Gew.-% bis 7 Gew.-%, mehr bevorzugt von 0,2 Gew.-% bis 5 Gew.-%, noch mehr bevorzugt von 0,3 Gew.-% bis 3,5 Gew.-% der Zusammensetzung umfasst.

7. Zahncremezusammensetzung nach einem der vorstehenden Ansprüche, wobei ein Zinkion aus der Zinkionenquelle ein Niveau von 400 Teile pro Million (ppm) bis 20.000 ppm, vorzugsweise 500 Teile pro Million (ppm) bis 15.000 ppm, vorzugsweise von 1.000 ppm bis 6.500 ppm, alternativ von 2.000 ppm bis 3.000 ppm umfasst.

8. Zahncremezusammensetzung nach einem der vorstehenden Ansprüche, wobei das Gewichtsverhältnis der Zinkionenquelle zu dem Copolymer aus Maleinsäureanhydrid und Methylvinylether von 1 : 4 bis 4 : 1; vorzugsweise von 1 : 3 bis 3 : 1; mehr bevorzugt von 1 : 2 bis 2 : 1 beträgt.

9. Zahncremezusammensetzung nach einem der vorstehenden Ansprüche, wobei das Pyrophosphat aus der Gruppe ausgewählt ist, bestehend aus Calciumpyrophosphat, Tetranatriumpyrophosphat, Dinatriumpyrophosphat, Tetrakaliumpyrophosphat und Kombinationen davon; vorzugsweise das Pyrophosphat Tetranatriumpyrophosphat ist.

10. Zahncremezusammensetzung nach Anspruch 9, wobei das Pyrophosphat von 0,1 Gew.-% bis 15 Gew.-%, vorzugsweise von 0,5 Gew.-% bis 8 Gew.-%, mehr bevorzugt von 0,9 Gew.-% bis 6 Gew.-%, noch mehr bevorzugt von 1 Gew.-% bis 5 Gew.-% der Zusammensetzung beträgt.

11. Zahncremezusammensetzung nach Anspruch 9 oder 10, wobei die Zinkionenquelle Zinkchlorid ist und wobei das Copolymer aus Maleinsäureanhydrid und Methyl-Copolymer 2-Butendisäure(2Z)-Polymer mit Methoxyethen ist.

12. Zahncremezusammensetzung nach einem der vorstehenden Ansprüche, wobei die Zinkionenquelle und das Copolymer aus Maleinsäureanhydrid und Methylvinylether einen Komplex ausbilden, vorzugsweise wobei das Copolymer aus Maleinsäureanhydrid und Methylvinylether hydrolysiert ist.

13. Zahncremezusammensetzung nach einem der vorstehenden Ansprüche, wobei die Fluoridionenquelle Natriummonofluorphosphat ist; vorzugsweise die Zusammensetzung von 0,2 Gew.-% bis 1,5 Gew.-%, vorzugsweise von 0,5 Gew.-% bis 1 Gew.-%, mehr bevorzugt von 0,6 Gew.-% bis 0,9 Gew.-% der Zusammensetzung das Natriummonofluorphosphat umfasst.

14. Zahncremezusammensetzung nach einem der vorstehenden Ansprüche, wobei das calciumhaltige Schleifmittel 27 Gew.-% bis 37 Gew.-% der Zusammensetzung das Calciumcarbonat umfasst.

15. Zahncremezusammensetzung nach einem der vorstehenden Ansprüche, wobei der pH-Wert über 8,1, vorzugsweise über dem pH-Wert 8,5, mehr bevorzugt über dem pH-Wert 9, alternativ von pH-Wert 8,5 bis pH-Wert 10,5 liegt.

16. Zahncremezusammensetzung nach einem der vorstehenden Ansprüche, wobei die Zusammensetzung eine einphasige Zusammensetzung ist.

## Revendications

1. Composition dentifrice comprenant :
(a) 50 % à 60 %, en poids de la composition, d'eau ;
(b) 27 % à 47 %, en poids de la composition, d'un abrasif contenant du calcium dans laquelle l'abrasif contenant du calcium comprend du carbonate de calcium ;
(c) 0,0025 % à 2 %, en poids de la composition, d'une source d'ion fluorure ;
(d) 0,01 % à 10 %, en poids de la composition, d'une source d'ion zinc ;
(e) 0,001 % à 5 %, en poids de la composition, d'un copolymère d'anhydride maléique et d'éther méthylvinylique ; et
(f) un polyphosphate, dans laquelle le polyphosphate est un pyrophosphate ; et
dans laquelle ladite composition a un pH supérieur à 8.

2. Composition dentifrice selon la revendication 1, dans laquelle le copolymère d'anhydride maléique et d'éther méthylvinylique, comprend de 0,01 % à 4 %, de préférence de 0,1 % à 3 %, plus préférablement de 0,2 % à 2 %, encore plus préférablement de 0,3 % à 1 %, en poids de la composition.

3. Composition dentifrice selon l'une quelconque des revendications précédentes, dans laquelle le copolymère d'anhydride maléique et d'éther méthylvinylique a une viscosité spécifique de 1 à 5, de préférence de 2 à 4,5, (selon le procédé décrit ici).

4. Composition dentifrice selon l'une quelconque des revendications précédentes, dans laquelle le copolymère d'anhydride maléique et d'éther méthylvinylique est le copolymère du polymère d'acide 2-butènedioïque (2Z)- avec du méthoxyéthène.

5. Composition dentifrice selon l'une quelconque des revendications précédentes, dans laquelle la source d'ion zinc est choisie dans le groupe constitué de citrate de zinc, lactate de zinc, tartrate de zinc, pyrophosphate de zinc, maléate de zinc, chlorure de zinc, et des combinaisons de ceux-ci ; de préférence la source d'ion zinc est du chlorure de zinc.

6. Composition dentifrice selon l'une quelconque des revendications précédentes, dans laquelle la source d'ion zinc comprend de 0,1 % à 7 %, plus préférablement de 0,2 % à 5 %, encore plus préférablement de 0,3 % à 3,5 %, en poids de la composition.

7. Composition dentifrice selon l'une quelconque des revendications précédentes, dans laquelle un ion zinc, de la source d'ion zinc, comprend un niveau de 400 parties par million (ppm) à 20 000 ppm, de préférence 500 parties par million (ppm) à 15 000 ppm, de préférence de 1 000 ppm à 6 500 ppm, en variante de 2 000 ppm à 3 000 ppm.

8. Composition dentifrice selon l'une quelconque des revendications précédentes, dans laquelle le rapport pondéral de la source d'ion zinc au copolymère d'anhydride maléique et d'éther méthylvinylique est de 1:4 à 4:1 ; de préférence de 1:3 à 3:1 ; plus préférablement de 1:2 à 2:1.

9. Composition dentifrice selon l'une quelconque des revendications précédentes, dans laquelle le pyrophosphate est choisi dans le groupe constitué de pyrophosphate de calcium, pyrophosphate tétrasodique, pyrophosphate disodique, pyrophosphate tétrapotassique, et des combinaisons de ceux-ci ; de préférence le pyrophosphate est le pyrophosphate tétrasodique.

10. Composition dentifrice selon la revendication 9, dans laquelle le pyrophosphate est de 0,1 % à 15 %, de préférence de 0,5 % à 8 %, plus préférablement de 0,9 % à 6 %, encore plus préférablement de 1 % à 5 %, en poids de la composition.

11. Composition dentifrice selon la revendication 9 ou 10, dans laquelle la source d'ion zinc est du chlorure de zinc et dans laquelle le copolymère d'anhydride maléique et de copolymère de méthyle est un polymère d'acide 2-butènedioïque (2Z)- avec du méthoxyéthène.

12. Composition dentifrice selon l'une quelconque des revendications précédentes, dans laquelle la source d'ion zinc et le copolymère d'anhydride maléique et d'éther méthylvinylique forment un complexe, de préférence dans laquelle le copolymère d'anhydride maléique et d'éther méthylvinylique est hydrolysé.

13. Composition dentifrice selon l'une quelconque des revendications précédentes, dans laquelle la source d'ion fluorure est du monofluorophosphate de sodium ; de préférence la composition comprend de 0,2 % à 1,5 %, de préférence de 0,5 % à 1 %, plus préférablement de 0,6 % à 0,9 %, en poids de la composition, du monofluorophosphate de sodium.

14. Composition dentifrice selon l'une quelconque des revendications précédentes, dans laquelle l'abrasif contenant du calcium comprend 27 % à 37 %, en poids de la composition, du carbonate de calcium.

15. Composition dentifrice selon l'une quelconque des revendications précédentes, dans laquelle le pH est supérieur à 8,1, de préférence supérieur à pH 8,5, plus préférablement supérieur à pH 9, en variante de pH 8,5 à pH 10,5.

16. Composition dentifrice selon l'une quelconque des revendications précédentes, dans laquelle la composition est une composition monophasique.
